# EUROPEAN PATENT APPLICATION

(11) **EP 2 400 465 A2**
(43) Date of publication of application: **28.12.2011**
(21) Application number: 11172987.7
(22) Date of filing: 26.11.2009
(51) Int. Cl.: G06T 19/00, A61B 5/055, A61B 6/03, G06T 15/00

(54) **Virtual endoscopy apparatus, method for driving thereof and medical examination apparatus**

(30) Priority: 10.09.2009 KR 20090085570; 10.09.2009 KR 20090085572
(62) Divisional of application: 09849282.0
(71) Applicant: Infinitt Healthcare Co., Ltd., Seoul 152-051 (KR)
(72) Inventor: Kwon, Sunggoo, 210-360 Gangwon-do (KR); Kim, Jinkook, 152-755 Seoul (KR); Yi, Jaeyoun, 136-824 Seoul (KR)
(74) Representative: Cabinet Plasseraud

(57) **Abstract**

The virtual endoscopy apparatus (110) comprises:
a data processor (120) generating virtual endoscopy data from input cross sectional image data of an inspection subject; and
a display unit (130) displaying images according to the virtual endoscopy data in a single screen.

The images displayed according to the virtual endoscopy data comprise at least one virtual endoscopy image and at least one reference image. The data processor (120) is configured to capture pictures of inside of the inspection subject while controlling a virtual camera to rotate around a movement path inside the inspection subject, and configured to generate the virtual endoscopy data by reconfiguring sub-areas which one of the picture is divided into, wherein each of the sub-areas corresponds to a predetermined specific angle.

## Description

### Technical Field

The present invention is related to a virtual endoscopy apparatus, method for driving thereof, and medical examination apparatus.

### Background Art

An endoscope is an instrument used for examining the health of the stomach, colon, and so on.

An endoscope is inserted directly into the inside of internal organs of a patient to examine the health of the internal organs.

In doing so, a patient is exposed to foreign body sensation, pain, etc. Moreover, internal organs of a patient may be injured during endoscopy.

To remedy the problem above, as a method for providing a three dimensional image by using a series of tomography images, a virtual endoscopy apparatus is being developed, where the apparatus emulates an actual endoscope, providing images which look like the images taken from an actual endoscope.

### Disclosure of Invention

### Technical Solution

In one aspecct, there is a virtual endoscopy apparatus comprising a data processor generating volume data expressed by a three dimensional position function by using contiguous cross sectional image data of an inspection subject and based on the volume data, generating virtual endoscopy data of inside of the inspection subject, and a display unit displaying virtual endoscopy images according to the virtual endoscopy data, wherein the data processor taking pictures of inside of the inspection subject while rotating a virtual camera around a movement path inside the inspection subject and generating the virtual endoscopy data at the same angle.

The virtual camera rotates perpendicular to the movement path.

Field of view of the virtual camera is between 60 degrees and 120 degrees.

The virtual camera rotates around the movement path and at the same time, proceeds along the movement path.

The virtual camera rotates while its forward movement is not performed.

Rotation angle of the virtual camera is 360 degrees or above.

Movement path inside the inspection subject is a line connecting center points of the cross sectional images.

The cross sectional image data are input from at least one of a computed tomography (CT) device and a magnetic resonance imaging (MRI) device.

The display unit displays the virtual endoscopy images obtained during rotation of the virtual camera unfolded in a direction of rotation of the virtual camera.

Both ends of the single virtual endoscopy image displayed in the display unit are overlapped with each other.

The two contiguous virtual endoscopy images displayed in the display unit have an overlapping part.

The virtual camera has a one-way movement path.

In another aspecct, there is a method for driving a virtual endoscopy apparatus comprising inputting contiguous cross sectional image data about an inspection subject, by using the cross sectional image data, generating volume data expressed by a three dimensional position function, setting a movement path inside the inspection subject, rotating a virtual camera around the movement path, and based on the volume data, obtaining virtual endoscopy images of inside of the inspection subject, and outputting the obtained virtual endoscopy images after reconfiguring according to the same angular distance, and displaying the virtual endoscopy images reconfigured according to the same angular distance.

In another aspecct, there is a virtual endoscopy apparatus comprising a data processor generating virtual endoscopy data from input cross sectional image data of an inspection subject, and a display unit displaying images according to the virtual endoscopy data in a single screen, the images according to the virtual endoscopy data comprising at least one virtual endoscopy image and at least one reference image, wherein the at least one virtual endoscopy image is obtained as a virtual camera takes pictures of the inspection subject while rotating around a movement path inside the inspection subject and obtained data of the inspection subject are reconfigured according to the same angle.

The data processor generates volume data expressed by a three dimensional position function by using contiguous cross sectional image data about the inspection subject and based on the volume data, generates virtual endoscopy data of inside of the inspection subject.

The reference image includes at least one of X-Y plane image, Y-Z plane image, and X-Z plane image of the inspection subject.

The reference image includes an image about the movement path of the virtual camera.

An image about the movement path displays a movement path about a part of the inspection subject.

An image about the movement path displays the entire movement path about the inspection subject.

If information of any image among at least one virtual endoscopy image and at least one reference image displayed together on the display unit is changed, information of at least one image among remaining images is changed in association therewith.

If an arbitrary first part is selected in an image from at least one virtual endoscopy image and at least one reference image displayed together on the display unit, at least one image among remaining images displays a part corresponding to the first part.

At least one image from among the at least one virtual endoscopy image and the at least one reference image is magnified or reduced according to a command input from outside.

At least one image from among the at least one virtual endoscopy image and the at least one reference image is displayed in the form of slideshow.

At least one image from among the at least one virtual endoscopy image and the at least one reference image is displayed in the form of a panoramic image.

If an arbitrary first part is designated as an interested part in at least one image among the at least one virtual endoscopy image and the at least one reference image, a part corresponding to the first part in at least one image among the remaining images is also designated as an interested part.

Images about the interested part are stored in memory.

If a loading command is input for the interested part, images about the interested part stored in the memory are displayed.

In another aspecct, there is a method for driving a virtual endoscopy apparatus comprising inputting contiguous cross sectional image data about an inspection subject, generating volume data expressed by a three dimensional position function by using the cross sectional image data, based on the volume data, generating virtual endoscopy data of the inspection subject, and displaying at least one virtual endoscopy image and at least one reference image according to the virtual endoscopy data, wherein the at least one virtual endoscopy image is obtained as a virtual camera takes pictures of the inspection subject while rotating around a movement path inside the inspection subject and obtained data of the inspection subject are reconfigured according to the same angle.

The method further comprise if information of any image among at least one virtual endoscopy image and at least one reference image displayed together on the display unit is changed, changing information of at least one image among remaining images in association therewith.

### Brief Description of Drawings

FIGs. 1 to 6 illustrate the structure and operations of a medical examination apparatus and virtual endoscopy apparatus according to the present invention;

FIGs. 7 to 9 illustrate embodiments according to the present invention and comparative examples;

FIGs. 10 to 12 illustrate a method for reconfiguring virtual endoscopy images;

FIGs. 13 to 16 illustrate an example where both a virtual endoscopy image and a reference image are displayed together on a screen;

FIGs. 17 to 19 illustrate a reference image displaying a movement path of a virtual camera;

FIGs. 20 to 24 illustrate an example of another function of a virtual endoscopy apparatus according to the present invention;

FIGs. 25 and 26 are diagrams for illustrating a rotation axis of a virtual camera;

FIG. 27 illustrates the motion of a virtual camera;

FIGs. 28 to 30 illustrate the field of view (FOV) of a virtual camera;

FIGs. 31 and 32 illustrate rotation angle of a virtual camera; and

FIGs. 33 and 34 illustrate movement distance of a virtual camera.

### Mode for the Invention

Hereinafter, preferred embodiments of the present invention will be described in detail with reference to appended drawings.

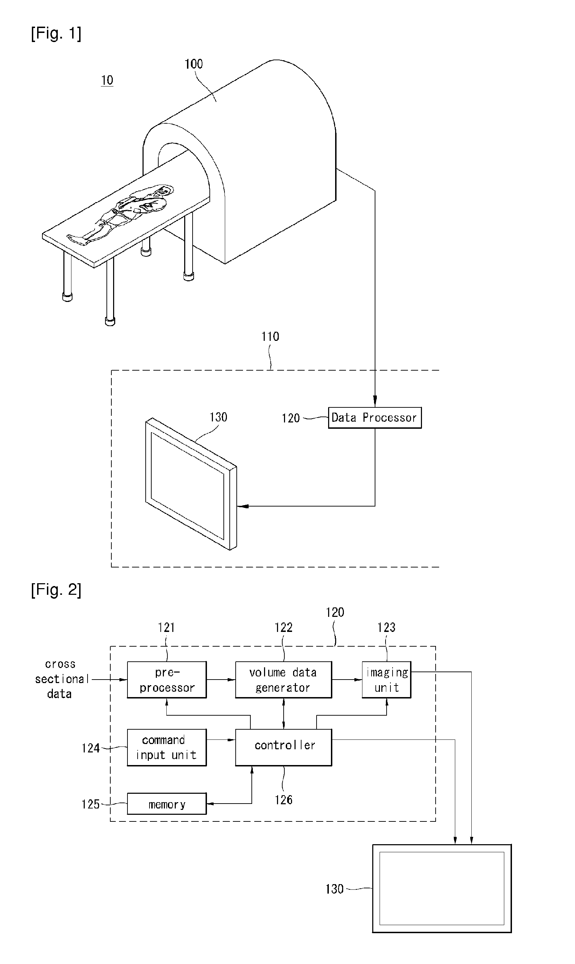

FIGs. 1 to 6 illustrate the structure and operations of a medical examination apparatus and virtual endoscopy apparatus according to the present invention.

First, with reference to FIG. 1, a medical examination apparatus 10 according to the present invention comprises a cross sectional image generation unit 100 and a virtual endoscopy apparatus 110.

A cross sectional image generation unit 100 can generate contiguous cross sectional image data of an inspection subject.

In addition, a virtual endoscopy apparatus 110 can generate and display a virtual endoscopy image from cross sectional image data of a prescribed inspection subject such as the stomach and colon of a human, generated by a cross sectional image generation unit 100.

Preferably, a virtual endoscopy apparatus 110 can comprise a data processor 120 which by using cross sectional image data generated by a cross sectional image generation unit 100, generates volume data expressed by a three dimensional position function and based on the volume data, takes pictures of the inside of an inspection subject while rotating a virtual endoscopy camera around a movement path inside the inspection subject and generates virtual endoscopy data at the same angle; and a display unit 130 which displays a virtual endoscopy image according to virtual endoscopy data and a reference image in one screen.

In addition, a cross sectional image generation unit 100 can be preferably either a computer tomography (CT) device or a magnetic resonance imaging (MRI) device which can capture cross sectional images of a prescribed inspection subject.

Also, as shown in FIG. 2, a data processor 120 can comprise a pre-processor 121, a volume data generator 122, an imaging unit 123, a command input unit 124, memory 125, and a controller 126.

A pre-processor 121 can process cross sectional image data of an inspection subject input from a cross sectional image generation unit 100 through interpolation, segmentation, and so on.

Interpolation is a process for increasing image resolution, which obtains differences of the previous and the next image from a current image in a contiguous image sequence and interpolates the difference images.

Segmentation is a process needed for generating three dimensional volume data, which sets a threshold value corresponding to the image intensity of an interested part and filters out those values below the threshold value.

A volume data generator 122 generates three dimensional volume data by using a rendering technique from cross sectional image data generated by a cross sectional image generator 100. Preferably, a volume data generator 122 can generate three dimensional volume data from image data processed by a pre-processor 121 by using a volume rendering technique.

An imaging unit 123, based on volume data generated by a volume data generator 122, can take pictures of the inside of an inspection subject by rotating a virtual endoscopy camera around a movement path inside the inspection subject.

Image data of the inside of an inspection subject photographed by an imaging unit 123 as described above can be output as virtual endoscopy data according to the same viewing angle.

A command input unit 124 feeds control commands input from the outside (e.g., from a user) to a controller 126 and thus enables the controller 126 to control the operations of a data processor 120.

Memory 125 can store various types of data such as captured images and information about an interested part.

Meanwhile, during photographing, a virtual camera can move along a movement path prescribed inside an inspection subject.

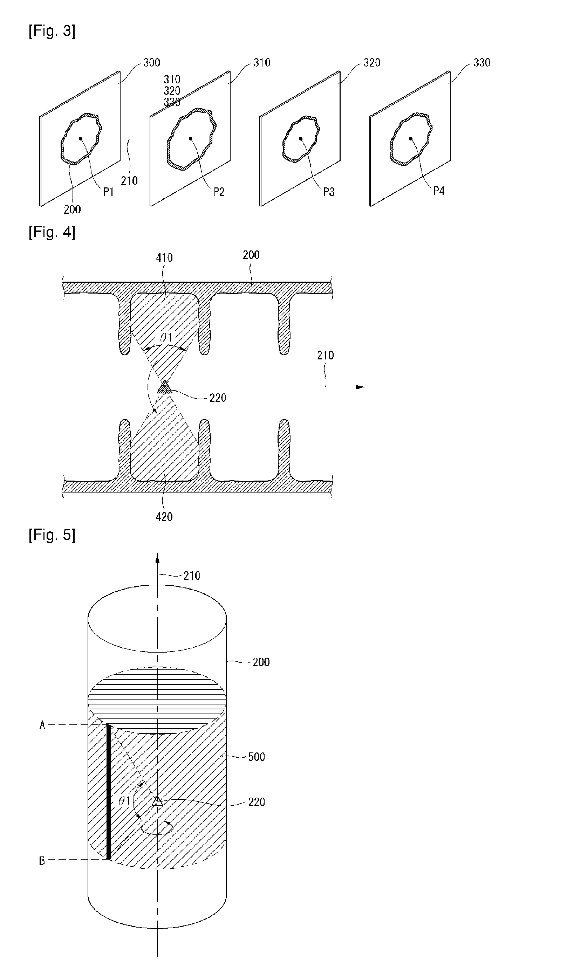

Also, as shown in FIG. 3, the movement path prescribed inside an inspection subject can be a line connecting center points P1∼P4 of cross sectional images 300-330 of the inspection subject 200 output by a cross sectional image generator 100.

A movement path prescribed inside an inspection subject is not limited to a line connecting the center points P1~P4 of cross sectional images 300~330. In some cases, it is equally allowed for the movement path not to pass at least one center point P1~P4 among cross sectional images 300~330.

A controller 126 can control the process of generating virtual endoscopy data.

According to the control of a controller 126, virtual endoscopy data output by an imaging unit 123 can be transmitted to a display unit 130; thereafter, the display unit 130 can display virtual endoscopy images.

In what follows, the operation of a virtual endoscopy apparatus 110 described above will be described in more detail with reference to FIG. 4. Hereinafter, it is assumed that an inspection subject is the colony of a human. An inspection subject of interest in the present invention can be varied including stomach, bronchus, and so on as well as the colony of a human.

With reference to FIG. 4, a virtual camera 220 can take pictures of the inside of the colony 220 with a prescribed field of view (θ1) while rotating around a movement path 210 prescribed inside the colony 200. A virtual camera 220 can rotate with the same angular step. For example, a virtual camera 220 can take pictures of the inside of the colony 200 while rotating 60 degrees for each step, performing six angular steps in total for one complete revolution.

Accordingly, upper part 410 and lower part 420 of the inside of the colony 200 can be photographed together from a viewpoint of a virtual camera 220.

For example, as shown in FIG. 5, a virtual camera 220 can take pictures while making one complete rotation around a movement path 210 prescribed inside the colony 200 of a cylindrical shape.

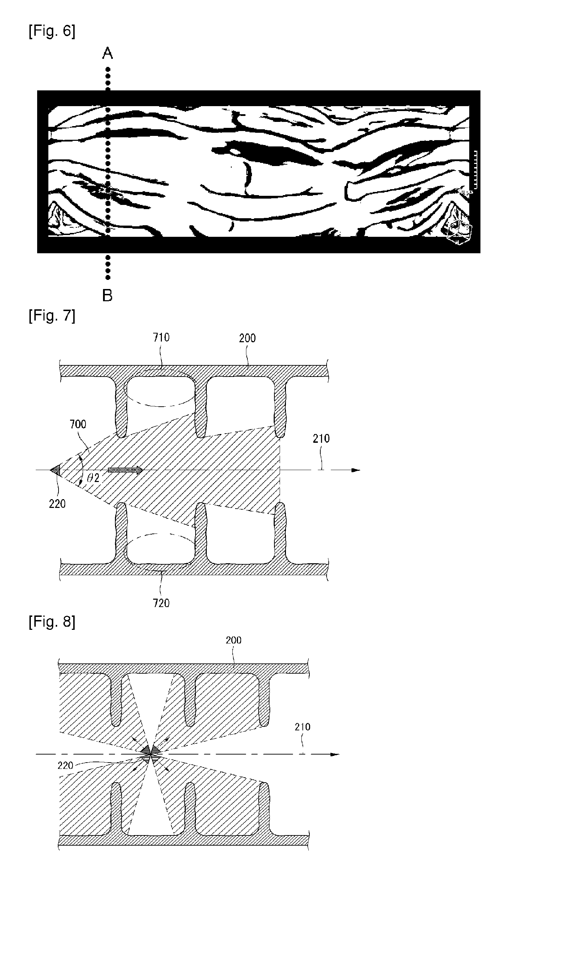

In addition, virtual endoscopy images according to virtual endoscopy data captured as shown in FIG. 5 can be displayed unfolded in a direction crossing a rotational axis 210 of a virtual camera 220 as shown in FIG. 6.

As shown in FIG. 6, if virtual endoscopy images captured by a virtual camera 220 are displayed unfolded in the direction of rotation of a virtual camera 220, inspection of the inner wall of the colony 200 can become more convenient.

For example, it can be considered important for colonography whether a polyp with a diameter of more than 6 mm has been formed on the inner wall of internal organs. Such a polyp usually has the shape of a circular protuberance. Polyps in the colony can be formed between the folds; it can be difficult to detect polyps between the folds of the colony.

On the other hand, as shown in FIGs. 5 and 6, if the inner wall of the colony 200 is captured by a virtual camera 220 rotating around a movement path 210 and virtual endoscopy images obtained are displayed unfolded in the direction of rotation of the virtual camera 220, gaps between the folds of the colony 200 can be more closely inspected and thus distortion of virtual endoscopy images can be reduced, by which detection of polyps can be made easier.

Accordingly, accuracy and speed of diagnosis can be improved.

In addition, since a virtual camera 220 takes pictures while rotating around a movement path 210, the virtual camera 220 is allowed to have a one-way movement path 210. Accordingly, time for taking pictures can be reduced.

FIGs. 7 to 9 illustrate embodiments according to the present invention and comparative examples;

First, FIG. 7 illustrates a method according to a first comparative example.

In a first comparative example, a virtual camera 220 moves straight ahead in a direction of the arrow along a movement path 210 prescribed inside the colony 200 and takes pictures of the inside of the colony 200 with a prescribed field of view θ2.

In a first comparative example, it is unavoidable that the area 700 which can be captured by a virtual camera 220 is very limited. The reason of the above is that since a virtual camera 220 takes pictures while moving straight ahead, areas 710, 720 not belonging to the field of view of the virtual camera 220 are generated.

Therefore, for the case of displaying virtual endoscopy images obtained by a method according to a first comparative example, it is unlikely to find polyps from the displayed images. Moreover, the displayed images suffer from distortion which makes diagnostic accuracy deteriorates.

Meanwhile, in the first comparative example, a virtual camera 220 can be made to move along a round-trip path to increase detection rate and diagnostic accuracy. In other words, a virtual camera 220 takes pictures of the colony 200 while moving along a movement path 210 in the direction of the arrow and then moving in the opposite direction of the arrow.

In that case, too, however, since a virtual camera 220 takes pictures of the colony 200 while moving straight ahead, areas not belonging to the field of view of the virtual camera 220 exist. Accordingly, distortion in the screen still remains and photographing time can be elongated according to the round-trip of the virtual camera 220.

Next, FIG. 8 illustrates a method according to a second comparative example.

As shown in FIG. 8, in the second comparative example, a virtual camera 220 can take pictures simultaneously from a plurality of directions while moving along a movement path 210.

For example, a virtual camera 220 can take pictures simultaneously from a total of six directions: upward, downward, left, right, forward, and backward direction.

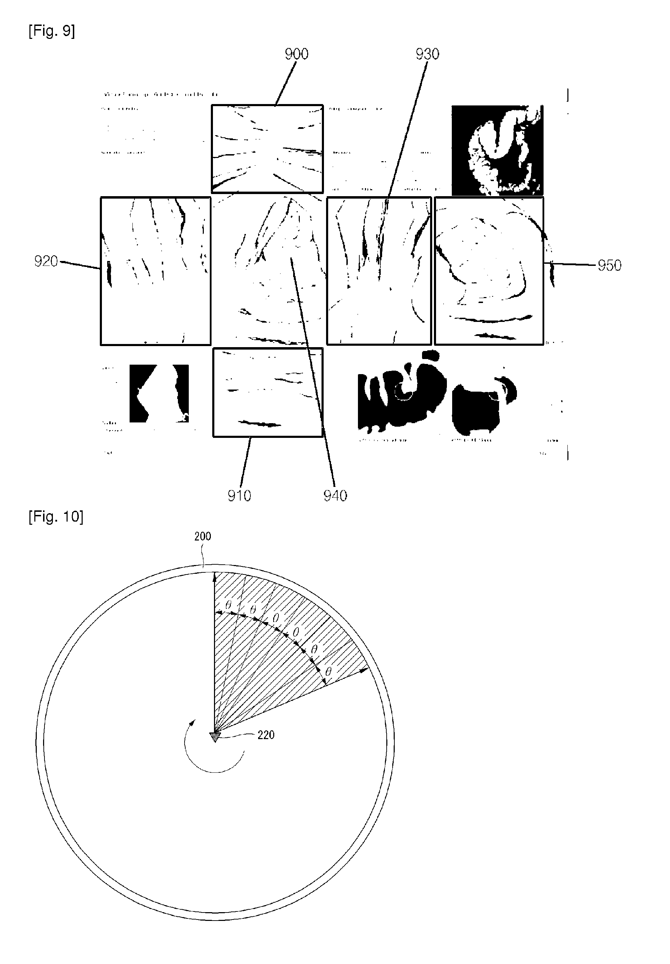

In addition, images taken from a method illustrated in FIG. 8 can be displayed unfolded at the same time as shown in FIG. 9.

For example, a first image 900 capturing in the upward direction of the colony 200 can be arranged in the upper part of a screen; a second image 910 capturing in the downward direction of the colony 200 in the lower part of the screen; a third image 920 capturing in the left direction of the colony 200 in the left of the screen; a fourth image 930 capturing in the right direction of the colony 200 in the right of the screen; a fifth image 940 capturing in the forward direction of the colony 200 in the center of the screen; and a sixth image 950 capturing in the backward direction of the colony 200 in the right of the fourth image 930.

In such a case, since images taken from different directions are displayed in one screen, distortion may occur at the boundaries of the respective images 900-950. In this way, diagnostic accuracy can be deteriorated.

On the other hand, as shown in FIG. 6, if a virtual camera 220 takes pictures of the inside of the colony 200 while rotating around a movement path 210 and those pictures are displayed unfolded in the direction of rotation of the virtual camera 220, distortion of virtual endoscopy images can be reduced, thereby diagnostic accuracy being improved.

Therefore, in an embodiment according to the present invention, diagnostic accuracy can be increased more than the comparative example 1 and 2.

FIGs. 10 to 12 illustrate a method for reconfiguring virtual endoscopy images.

First, as shown in FIG. 10, in the present invention, a rotating virtual camera 220 takes pictures of the colony 200 and the data from the virtual endoscopy images can be reconfigured according to the same rotation angle. For example, a virtual camera 220 can take pictures as the camera rotates clockwise with an angular step of 6θ. Also, the image data obtained from the virtual camera 220 which rotates with an angular step of 6θ can be reconfigured in accordance with the value of θ. In other words, virtual endoscopy images taken by a virtual camera 220 are reconfigured respectively according to the same angle θ.

In this way, if a rotating virtual camera 220 takes pictures and the image data from the virtual endoscope are reconfigured according to the same angle, distortion at the boundaries of images can be suppressed. Accordingly, diagnostic accuracy can be increased a lot.

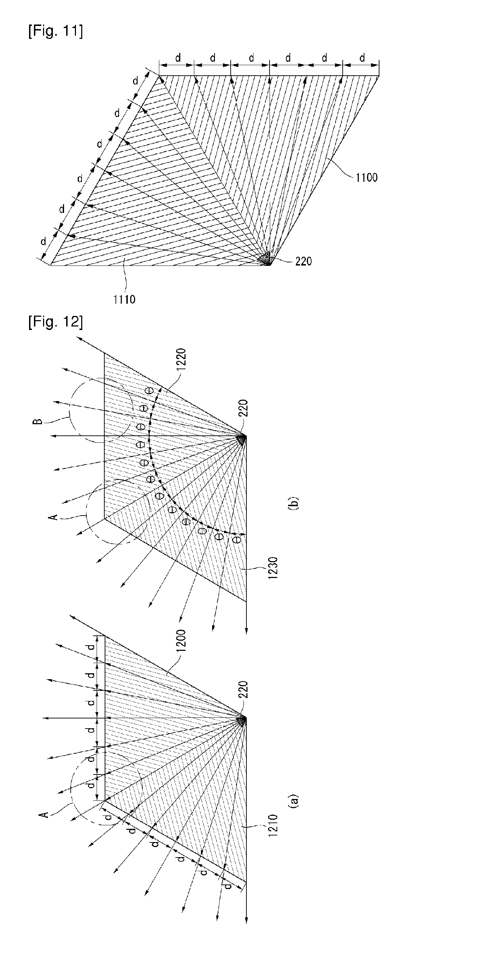

Meanwhile, different from the present invention, FIG. 11 illustrates an example of a method for reconfiguring a virtual camera 220 according to the same distance.

The method shown in FIG. 11 obtains images by reconfiguring image data of a virtual endoscope to be divided by equal distances in an image panel from each other.

In other words, when a virtual camera 220 takes pictures of a first area 1100, the first area 1100 is divided into a plurality of sub-areas by a prescribed distance d and images are captured for the individual sub-areas. Also, if the virtual camera 220 takes pictures of a second area 1110, the second area 1110 is divided into a plurality of sub-areas by a prescribed distance d and images are taken for the individual sub-areas.

Thus, virtual endoscopy images of the inside of the colony 200 can be obtained in such a way that virtual endoscopy data are reconfigured according to the same distance by using a method shown in FIG. 11.

If a method as shown in FIG. 11 is used, however, distortion can be increased at the boundaries of images.

The above situation can be explained more clearly with reference to FIG. 12.

If image data of a virtual endoscope obtained by a virtual camera 220 are reconfigured according to the same distance d as shown in FIG. 12, the boundary between the image of a first area 1200 and the image of a second area 1210 becomes more magnified, thereby introducing image distortion.

The reason of the above situation is that if virtual endoscopy data are reconfigured according to the same distance d, the boundary area A between a first area 1200 and a second area 1210 can be more closely observed and thus, the corresponding area A becomes more magnified when individual images are displayed on a screen.

On the other hand, FIG. 12 (b) illustrates the case of reconfiguring virtual endoscopy data by the same angular distance.

In the case of FIG. 12 (b), the boundary area A between a first area 1220 and a second area 1230 and a central area B can be viewed with actually the same image quality from a viewpoint of a virtual camera 220.

Accordingly, if image data of a virtual endoscope reconfigured according to a method of FIG. 12 (b) are displayed, image distortion can be reduced.

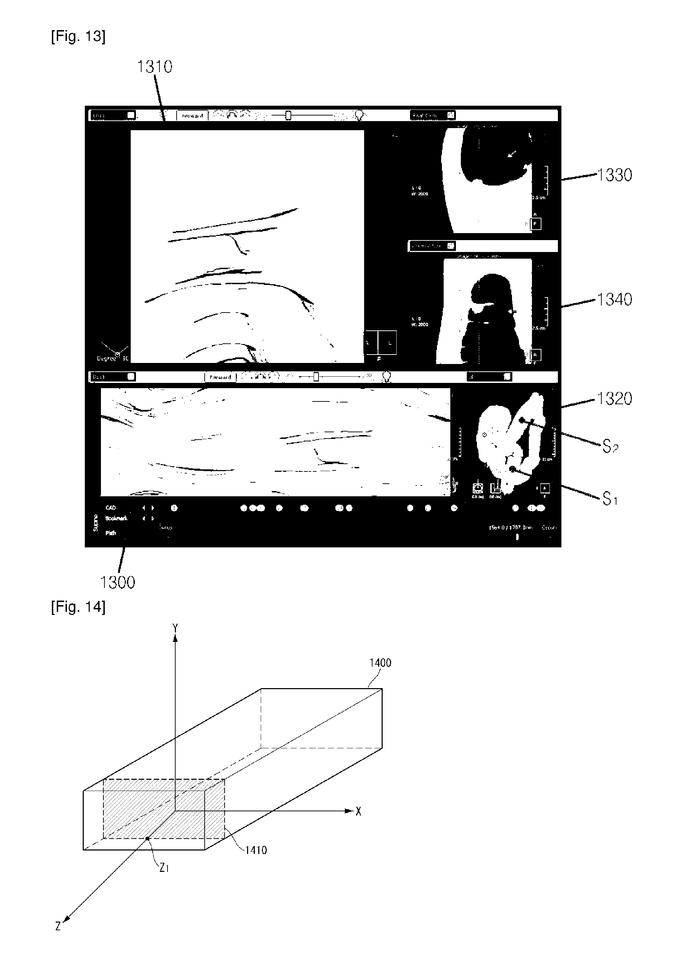

FIGs. 13 to 16 illustrate an example where both a virtual endoscopy image and a reference image are displayed together on a screen.

First, as shown in FIG. 13, at least one image among those displayed in a screen can include virtual endoscopy image 1310, 1300.

Also, at least one image among at least one virtual endoscopy image 1310, 1300 has been made in such a way that a virtual camera takes pictures of an inspection subject while rotating around a movement path and virtual endoscopy data obtained are reconfigured by the same angular distances.

In what follows, band view image 1300 is used to indicate those virtual endoscopy images 1300 which have been generated as a virtual camera takes pictures of an inspection subject while rotating around a movement path and the virtual endoscopy data obtained are reconfigured by the same angular distances. The band view image 1300 has been described in detail above; repeated descriptions are omitted in what follows.

Another virtual endoscopy image 1310 shown in FIG. 13 can correspond to the image of an inspection subject obtained by using a method described in FIG. 7.

In this way, if a virtual endoscopy image 1310 of a different type from a band view image 1300 is displayed together with the band view image 1300, an inspection subject can be observed in various view points, diagnostic accuracy can be improved and the time needed for examination can be reduced further.

Also, reference images 1320, 1330, 1340 can be displayed on a screen together with a band view image 1300.

Such reference images 1320, 1330, 1340 provide an observer with more detailed information and thus, diagnostic efficiency can be improved.

Among the reference images 1320, 1330, 1340, at least one image can correspond to an image 1320 where a movement path of a virtual camera is indicated. A reference image with an identification number 1320 is called a first reference image.

Looking at a first reference image 1320, it can be observed that an inspection subject, colony is displayed and a movement path of a virtual camera is indicated by lines at the central area of the colony.

Also, at least one image among reference images 1320, 1330, 1340 can correspond to an X-Y plane image of an inspection subject. For example, as shown in FIG. 14, a reference image can correspond to an axial image 1410 which has the same z-coordinate value of the inspection subject 1400.

Also, at least one image among reference images 1320, 1330, 1340 can correspond to a Y-Z plane image of an inspection subject. For example, a reference image can correspond to a sagittal image 1420 which has the same x-coordinate value of the inspection subject 1400.

Likewise, at least one image among reference images 1320, 1330, 1340 can correspond to an X-Z plane image of an inspection subject. For example, a reference image can correspond to a coronal image 1430 which has the same y-coordinate value of the inspection subject 1400.

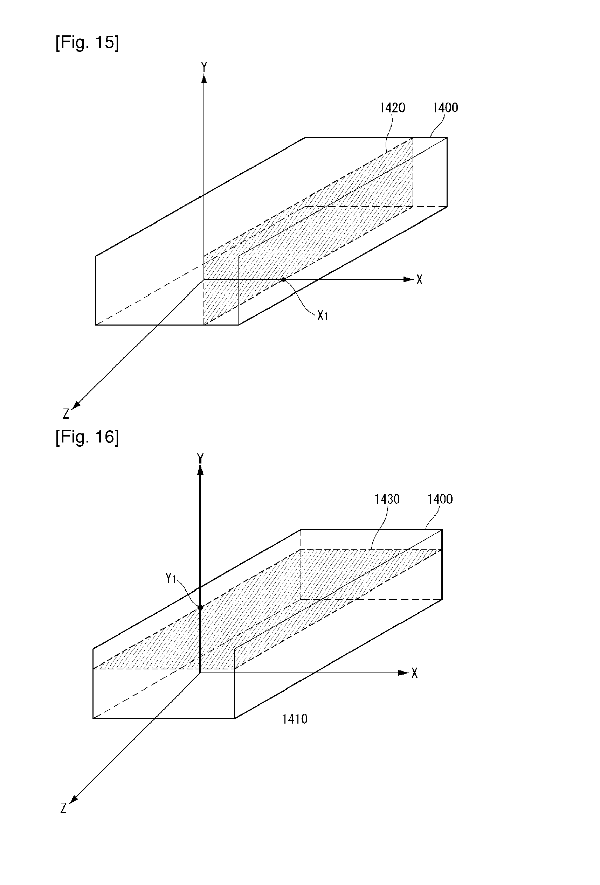

As shown in FIG. 13, if horizontal direction of another virtual endoscopy image 1310 is assumed to be x-axis and vertical direction is assumed to be y-axis, a second reference image 1330 among reference images 1320, 1330, 1340 can correspond to a coronal image 1430 which has the same y-coordinate value as shown in FIG. 16, while a third reference image 1340 among reference images 1320, 1330, 1340 can correspond to a sagittal image 1420 which has the same x-coordinate value as shown in FIG. 15.

If information of any image among at least one virtual endoscopy image 1310, 1300 and at least one reference image 1320, 1330, 1340 displayed together on a display unit is changed, it is preferable that information of at least one image among remaining images is changed in association therewith.

For example, as shown in FIG. 13, if the user selects a first position S1 in a first reference image 1320 by using a command input means such as a mouse, a virtual endoscopy image 1300, 1310 is displayed to respond to the selection of the first position S 1 and a reference image 1320, 1330, 1340 is also displayed to respond to the selection of the first position S 1.

Afterwards, if the user selects a second position S2 in a first reference image 1320, a virtual endoscopy image 1320, 1310 and a reference image 1320, 1330, 1340 can be changed to an image corresponding to the second position S2 from an image corresponding to the first position S 1.

More specifically, if a command is input through a command input unit 124 shown in FIG. 2 indicating that a selected position is changed from a first position S 1 to a second position S2 in a first reference image 1320, a controller 126 confirms the command and according to the confirmation result, the controller 126 controls a virtual endoscopy image 1300, 1310 and a second and third reference image 1330, 1340 to display images corresponding to the second position S2.

In this way, since various images displayed on a screen are related to each other, the user can easily observe interested image parts by applying simple mouse operations quickly.

Also, in the present invention, the user can designate any part of an inspection subject as an interested part.

For example, the user can designate a first position S1 in a first reference image 1320 as an interested part.

In that case, a particular mark can be assigned to indicate the first position S 1 in the first reference image 1320 as an interested part.

Also, images corresponding to the first position S 1 from a second, third reference image 1330, 1340 and a virtual endoscopy image 1300, 1310 can also be stored in the memory as the data of an interested part.

In other words, if an arbitrary first part is designated as an interested part in at least one image among at least one virtual endoscopy image and at least one reference image, a part corresponding to the first part in at least one image among the remaining images can also be designated as an interested part.

Also, images corresponding to an interested part are stored in the memory.

Afterwards, a loading command can be input through a command input unit to check if the data correspond to an interested part designated previously by the user.

In this case, images stored in the memory corresponding to the interested part can be displayed in a display unit.

For example, if a first position S1 in a first reference image 1320 of FIG. 13 is designated as an interested part and the user loads data of the first position S 1 as an interested part, the data of a second, third reference image 1330, 1340 and a virtual endoscopy image 1300, 1310 corresponding to the first position S 1 are also loaded to be displayed in one screen.

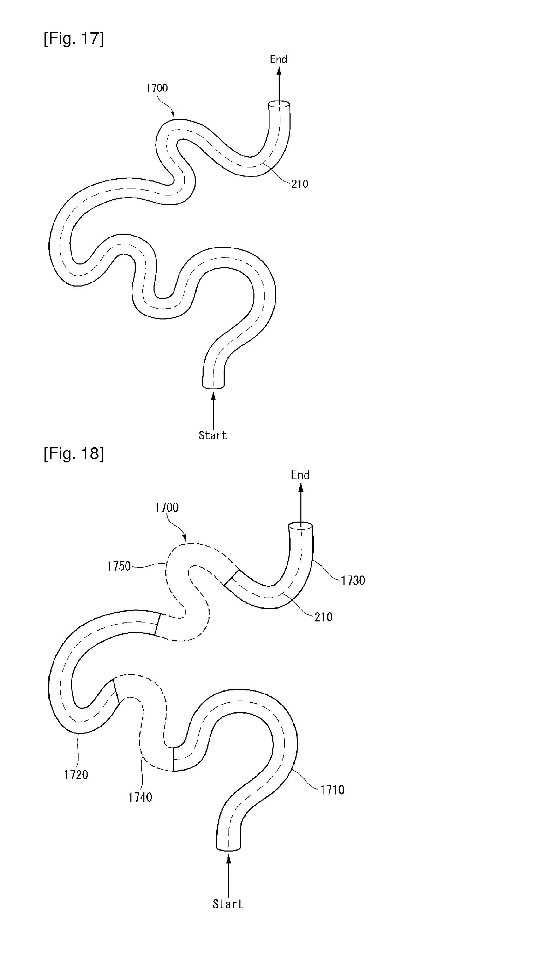

FIGs. 17 to 19 illustrate reference images indicating a movement path of a virtual camera.

First, with reference to FIG. 17, a reference image about a movement path can correspond to an image displaying the entire movement path 210 about an inspection subject 1700. For example, it is possible to display a movement path of a virtual camera covering the entire colony of a human.

In this case, since an observer can easily select an interested part for examination in a reference image, diagnostic efficiency can be improved.

On the other hand, as shown in FIG. 18, a reference image about a movement path can be an image displaying a movement path 210 for a part of an inspection subject 1700.

For example, movement paths for a first part 1710, a third part 1720, and a fifth part 1730 of an inspection subject 1700 can be displayed but the remaining second part 1740 and fourth part 1750 can be omitted.

In other words, an observer can select only a part desired for examination from an inspection object 1700 and display a movement path 210 thereon, thereby capturing only the selected part.

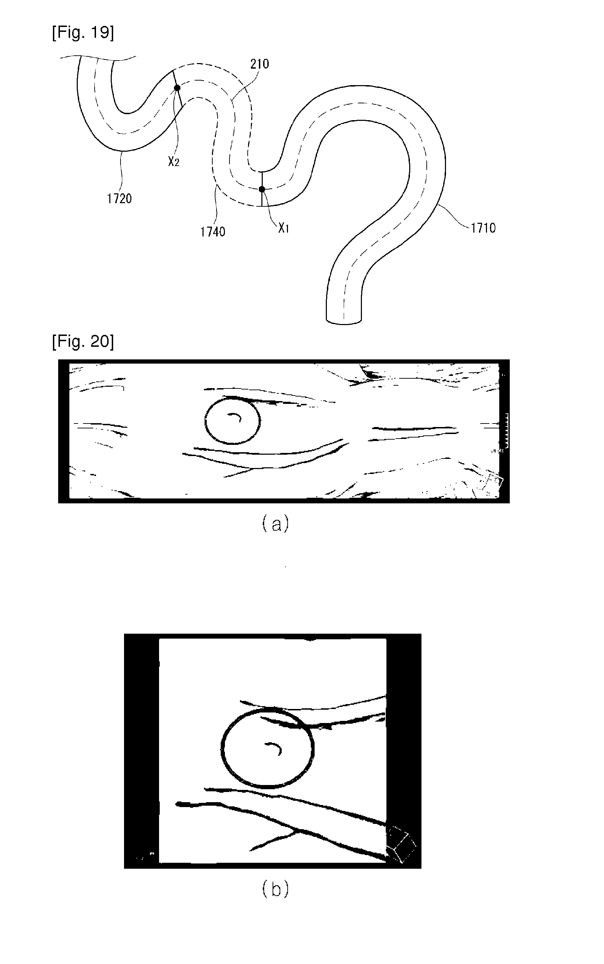

Alternatively, as shown in FIG. 19, a movement path 210 can additionally be recovered for the part where display of a movement path 210 has been omitted.

For example, while a second part 1740 is missing, the second part being located between a first part 1710 and a third part 1720, if the second part 1740 is desired to be recovered, an observer can select the end point X1 of the first part 1710 and the start point X2 of the third part 1720.

Afterwards, an observer can input a command for recovering the part located between the end point X1 of the first part 1710 and the start point X2 of the third part 1720; in this case, the second part 1740 is recovered along with a movement path 210 displayed on the second part 1740.

FIGs. 20 to 24 illustrate an example of another function of a virtual endoscopy apparatus according to the present invention.

First, as shown in FIG. 20, at least one image from among at least one virtual endoscopy image and at least one reference image can be magnified or reduced according to a command input from the outside. In other words, a virtual endoscopy apparatus according to the present invention provides a function of magnifying or reducing a desired part of a chosen image.

For example, as shown in FIG. 20(a), while a band view image is displayed, if an observer wants to magnify a part marked in (a), the observer can input a command to magnify the marked part.

Then, as shown in FIG. 20 (b), the selected part can be displayed magnified.

In this way, if a desired part is magnified, a close examination can be made possible and accordingly, diagnostic accuracy can be improved.

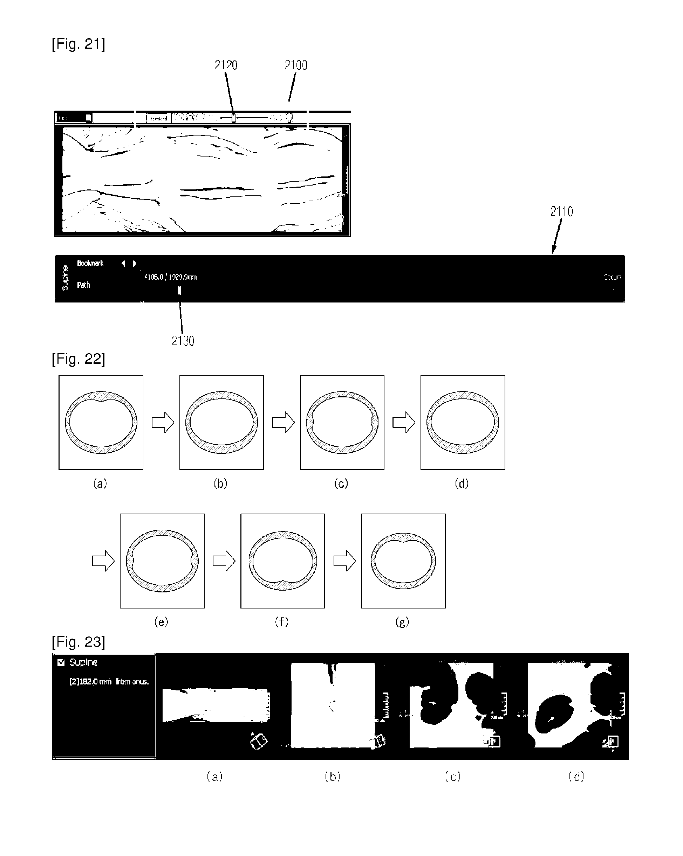

On the other hand, as shown in FIG. 21, at least one image from among at least one virtual endoscopy image and at least one reference image can be displayed in the form of a panoramic image.

For example, a scrollable functionality window 2100, 2110 can be put on a part of a band view image. An observer can move a scroll bar 2120, 2130 prepared in the functionality window 2100, 2110 by using a command input means such as a mouse. The band view image is then displayed continuously in the form of a panoramic image according to the input command of the observer.

On the other hand, as shown in FIG. 22, at least one image from among at least one virtual endoscopy image and at least one reference image can be displayed in the form of a slideshow.

For example, if a slideshow command is input, contiguous images a∼f are displayed sequentially with a prescribed time interval.

Alternatively, as shown in FIG. 23, any part of an image designated by an observer can be captured and stored in the memory.

For example, if an observer selects a first part S1 of a first reference image 1320 of FIG. 13 as an interested part, virtual endoscopy images a, b and reference images c, d corresponding to the first part S 1 can be captured.

In this way, captured images a∼d can be stored in the memory. At the same time, the captured images can be loaded according to a loading command from the user and displayed together.

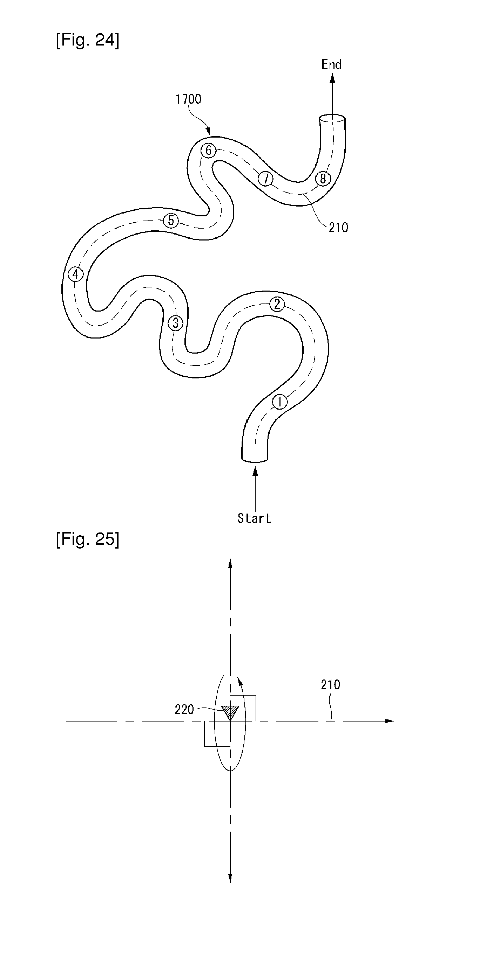

On the other hand, as shown in FIG. 24, an interested part designated by an observer can be displayed on a screen.

For example, as shown in FIG. 24, if a suspected part is found while examining a virtual endoscopy image of an inspection subject 1700, each individual part can be designated as an interested part and marked on a screen. FIG. 24 illustrates a case where the number of interested parts designated by an observer is eight in total and the interested parts are marked by ①②③④⑤⑥⑦⑧.

Afterwards, if an observer selects at least one mark for examination from among the marks displayed on a screen, a virtual endoscopy image or reference image corresponding to the selected mark can be displayed on a screen.

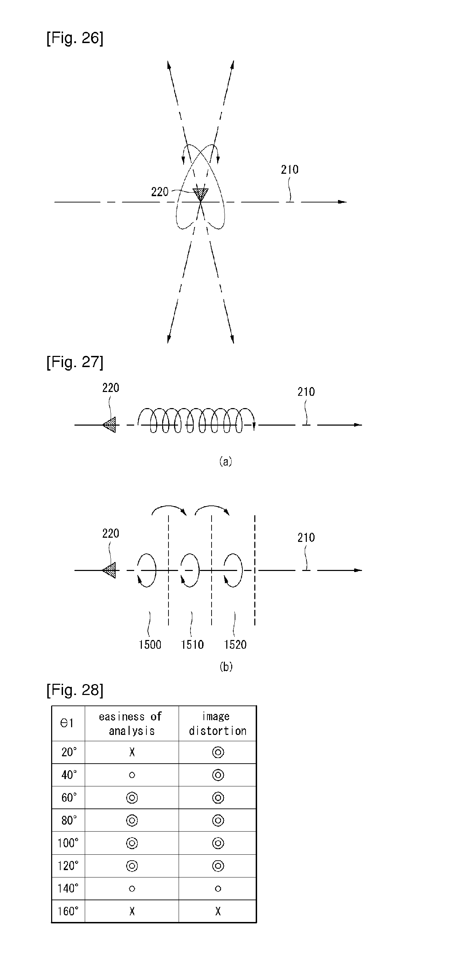

FIGs. 25 and 26 are diagrams for illustrating a rotation axis of a virtual camera.

First, FIG. 25 illustrates that a virtual camera 220 can rotate perpendicular to a movement path 210.

In addition, as shown in FIG. 26, a virtual camera 220 can rotate around a movement path 210 making an acute angle or obtuse angle with respect to the movement path 210.

In this way, if a virtual camera 220 rotates around a movement path 210 making an acute angle or obtuse angle with respect to the movement path 210, a much larger area can be captured with a single motion.

FIG. 27 illustrates the motion of a virtual camera.

With reference to FIG. 27, as shown in (a), a virtual camera 220 can rotate around a movement path 210 and at the same time, can proceed along the movement path 210. In this case, increasing the speed of a virtual camera 220 can be possible.

On the other hand, as shown in (b), a virtual camera 220 can rotate while its forward movement is not performed. For example, a virtual camera 220 can rotate in a first area 1500 taking pictures of an inspection subject and then move to a second area 1510 and a third area 1520 sequentially and rotate in the respective areas taking pictures of the inspection subject.

In the case of (b), it can be made simple to obtain virtual endoscopy images where distortion is kept to a minimum.

FIGs. 28 to 30 illustrate the field of view (FOV) of a virtual camera.

The data of FIG. 28 show judgment results about easiness of analyzing virtual endoscopy images and distortion found in the virtual endoscopy images when the field of view θ 1 of a virtual camera ranges from 20 degrees to 160 degrees.

To be specific, multiple observers analyzed virtual endoscopy images according to the field of view of a virtual camera and made respective judgments about easiness of analyzing virtual endoscopy images and distortion found therein; those judgment results have been assembled and passed through an assessment.

A mark X indicates very bad condition due to either difficulty in analyzing virtual endoscopy images or distortion found in the virtual endoscopy images. A mark O indicates satisfactory condition and the mark ⊚ indicates excellent condition.

With reference to FIG. 28, as for the distortion of virtual endoscopy images, distortion becomes most severe when the field of view of a virtual camera 220 is 160 degrees.

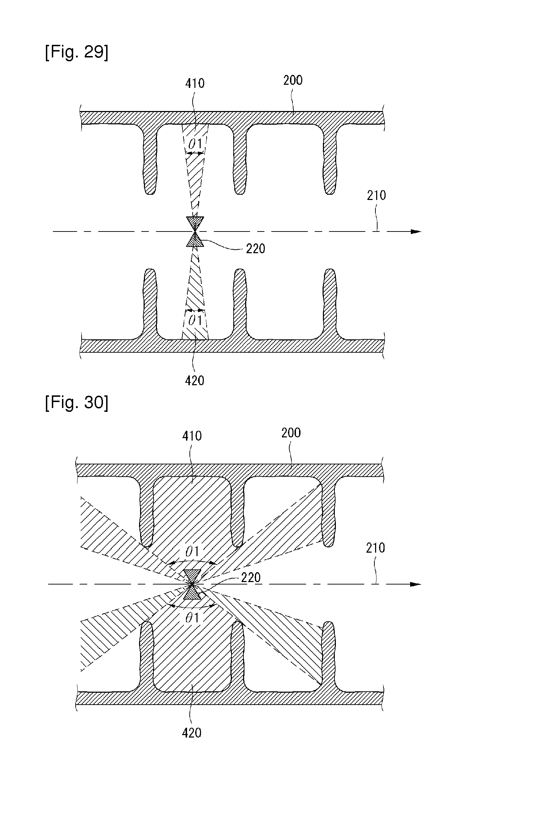

In this case, as shown in FIG. 30, since the field of view θ1 of a virtual endoscopy camera 220 is exceedingly large, some parts are not observed within the field of view θ1 of the virtual endoscopy camera 220 and accordingly, distortion arises in the virtual endoscopy images.

On the contrary, in terms of distortion of a virtual endoscopy image, if the field of view θ1 of a virtual camera 220 falls between 20 degrees and 120 degrees, image quality becomes highly satisfactory.

In this case, since virtually everything can be observed within the field of view θ1 of a virtual camera 220 as the field of view θ 1 of the virtual camera 220 is optimized, distortion in a virtual endoscopy image can be prevented.

Also, in terms of distortion in a virtual endoscopy image, if the field of view θ1 of a virtual camera 220 is 140 degrees, it is observed that image quality is satisfactory.

In that case, some parts may not be observed within the field of view θ1 of a virtual camera 220 but their overall effect is of little significance.

Meanwhile, in terms of easiness of analyzing a virtual endoscopy image, it can be seen that degree of easiness is very low if the field of view θ1 of a virtual camera 220 is 20 degrees.

In this case, as shown in FIG. 29, since the field of view θ1 of a virtual endoscopy camera 220 is excessively small, the number of images needed for diagnosis becomes excessively large. Accordingly, extremely long time may be needed for analyzing a virtual endoscopy image.

On the other hand, in terms of easiness of analyzing a virtual endoscopy image, if the field of view θ1 of a virtual camera 220 is between 60 degrees and 120 degrees, degree of easiness is quite satisfactory.

In this case, time needed for analyzing a virtual endoscopy image can be reduced as the field of view θ1 of a virtual camera 220 is optimized.

Meanwhile, in terms of easiness of analyzing a virtual endoscopy image, if the field of view θ1 of a virtual camera 220 is 140 degrees, degree of easiness is relatively satisfactory.

In this case, analysis for a boundary part between two neighboring images may not be easy since the part of the colony 200 displayed in a single image is large but its overall effect is of little significance.

On the other hand, in terms of easiness of analyzing a virtual endoscopy image, if the field of view θ1 of a virtual camera 220 is 160 degrees, degree of easiness becomes severely deteriorated.

In this case, since the field of view θ1 of a virtual camera 220 is excessively large, some parts may not be observed within the field of view θ1. Accordingly, distortion arises in the boundary area between neighboring two images, making analysis difficult.

Considering the data above, it is preferable for a virtual camera 220 to have a field of view angle between 60 to 120 degrees.

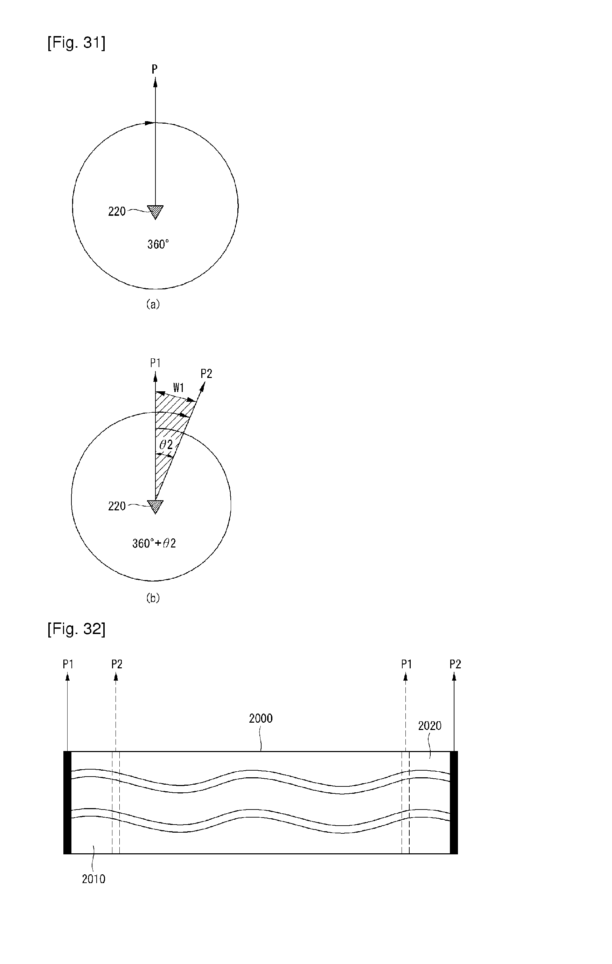

FIGs. 31 and 32 illustrate rotation angle of a virtual camera.

First, with reference to FIG. 31, rotation angle of a virtual camera 220 can be larger than 360 degrees as shown in (b). Likewise, rotation angle of the virtual camera 220 can effectively be 360 degrees as shown in (a).

As shown in (a), if the rotation angle of a virtual camera 220 makes an effective angle of 360 degrees, both ends of a single virtual endoscopy image displayed unfolded in the direction of rotation of the virtual camera 220 may coincide with each other. In other words, images at both ends of a single virtual endoscopy image are repeated.

On the other hand, if the effective angle of a virtual camera 220 is larger than 360 degrees as shown in (b), both ends of a single virtual endoscopy image displayed unfolded in the direction of rotation of the virtual camera 220 may be overlapped with each other. In other words, image at both ends of a single virtual endoscopy image include actually the same contents.

For example, as shown in FIG 32, a single virtual endoscopy image displayed unfolded in the direction of rotation of a virtual camera 220 can comprise a central image 2000, a first lateral image 2010 located at the leftmost end of the central image 2000, and a second lateral image 2020 located at the rightmost end of the central image 2000.

At this point, a first lateral image 2010 and a second lateral image 2020 are those images located between a first position P1 and a second position P2 and can be identical to each other.

In this way, if both ends of a single virtual endoscopy image are overlapped with each other, image distortion at the boundaries of both ends is prevented and thus diagnostic accuracy can be further increased.

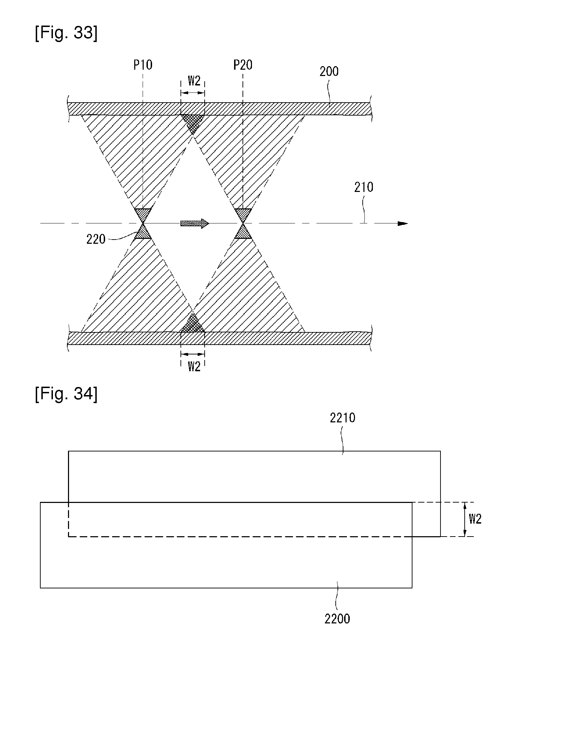

FIGs. 33 and 34 illustrate movement distance of a virtual camera.

First, with reference to FIG. 33, let us assume that a virtual camera 220 moves to a 20th position P20 and takes pictures after taking pictures at an arbitrary 10th position P 10.

In this case, as shown in FIG. 33, the field of view of a virtual camera 220 taking pictures at an arbitrary 10th position P10 can overlap with a part W1 of the field of view at a 20th position P20 which is on a continuous line originating from the 10th position.

Then, two contiguous images as shown in FIG. 34 can have an overlapping part W1. In this case, the two contiguous images can correspond to an image unfolded in the direction of rotation of a virtual camera 220.

In this way, if two contiguous images have an overlapping part W1, distortion in the boundaries of the two contiguous images can be prevented.

## Claims

1. A virtual endoscopy apparatus (110), comprising:
a data processor (120) generating virtual endoscopy data from input cross sectional image data of an inspection subject; and
a display unit (130) displaying images according to the virtual endoscopy data in a single screen,
**characterized in that** the images displayed according to the virtual endoscopy data comprises at least one virtual endoscopy image and at least one reference image, and
the data processor (120) is configured to capture pictures of inside of the inspection subject while controlling a virtual camera (220) to rotate around a movement path (210) inside the inspection subject, and configured to generate the virtual endoscopy data by reconfiguring sub-areas which one of the picture is divided into, wherein each of the sub-areas corresponds to a predetermined specific angle.

2. The apparatus of claim 1, wherein the data processor (120) generates volume data expressed by a three dimensional position function by using contiguous cross sectional image data about the inspection subject and based on the volume data, generates virtual endoscopy data of inside of the inspection subject.

3. The apparatus of claim 1, wherein the reference image includes at least one of X-Y plane image, Y-Z plane image, and X-Z plane image of the inspection subject.

4. The apparatus of claim 1, wherein the reference image includes an image about the movement path (210) of the virtual camera (220).

5. The apparatus of claim 4, wherein an image about the movement path (210) displays a movement path about a part of the inspection subject.

6. The apparatus of claim 4, wherein an image about the movement path (210) displays the entire movement path about the inspection subject.

7. The apparatus of claim 1, wherein if information of any image among at least one virtual endoscopy image and at least one reference image displayed together on the display unit is changed, information of at least one image among remaining images is changed in association therewith.

8. The apparatus of claim 7, wherein if an arbitrary first part is selected in an image from at least one virtual endoscopy image and at least one reference image displayed together on the display unit, at least one image among remaining images displays a part corresponding to the first part.

9. The apparatus of claim 1, wherein at least one image from among the at least one virtual endoscopy image and the at least one reference image is magnified or reduced according to a command input from outside.

10. The apparatus of claim 1, wherein at least one image from among the at least one virtual endoscopy image and the at least one reference image is displayed in the form of slideshow.

11. The apparatus of claim 1, wherein at least one image from among the at least one virtual endoscopy image and the at least one reference image is displayed in the form of a panoramic image.

12. The apparatus of claim 1, wherein if an arbitrary first part is designated as an interested part in at least one image among the at least one virtual endoscopy image and the at least one reference image, a part corresponding to the first part in at least one image among the remaining images is also designated as an interested part.

13. The apparatus of claim 12, wherein images about the interested part are stored in memory.

14. The apparatus of claim 13, wherein if a loading command is input for the interested part, images about the interested part stored in the memory are displayed.

15. A method for driving a virtual endoscopy apparatus, the method **characterized in that**:
inputting contiguous cross sectional image data about an inspection subject;
generating volume data expressed by a three dimensional position function by using the cross sectional image data;
generating, based on the volume data, virtual endoscopy data of the inspection subject; and
displaying at least one virtual endoscopy image and at least one reference image according to the virtual endoscopy data,
wherein the at least one virtual endoscopy image is obtained by controlling a virtual camera (220) to capture pictures of the inspection subject while controlling the virtual camera to rotate around a movement path (210) inside the inspection subject, and
wherein the obtained data of the inspection subject are generated by reconfiguring sub-areas which one of the picture is divided into, wherein each of the sub-areas corresponds to a predetermined specific angle.
